# EUROPÄISCHE PATENTSCHRIFT

(11) **EP 2 861 878 B1**
(45) Veröffentlichungstag und Bekanntmachung des Hinweises auf die Patenterteilung: **09.08.2017**
(21) Anmeldenummer: 12728406.5
(22) Anmeldetag: 15.06.2012
(51) Int. Cl.: F16C 11/10, F16M 11/06, F16M 11/20, B25J 19/00, B25J 17/02

(54) **BIDIREKTIONALES SCHWENKGELENK SOWIE EINE DAS SCHWENKGELENK IN MEHRZAHL UMFASSENDE HALTE- UND POSITIONIEREINRICHTUNG**
BIDIRECTIONAL SWIVEL JOINT AND DEVICE FOR HOLDING AND POSITIONING COMPRISING A PLURALITY OF SWIVEL JOINTS
ARTICULATION PIVOTANTE BIDIRECTIONALE ET DISPOSITIF DE RETENUE ET POSITIONNEMENT COMPRENANT UNE MAJORITÉ D'ARTICULATIONS

(43) Veröffentlichungstag der Anmeldung: 22.04.2015
(73) Patentinhaber: Klews, Peter-Michael, 21357 Barum (DE)
(72) Erfinder: AHRENS, Kerstin, 21436 Marschacht (DE); BEHR, Marko, 21395 Tespe (DE)
(74) Vertreter: Stork Bamberger Patentanwälte PartmbB
(86) Internationale Anmeldenummer: PCT/EP2012/002590
(87) Internationale Veröffentlichungsnummer: WO 2013/185791

(56) Entgegenhaltungen:
- DE-A1- 10 209 209
- DE-A1- 19 508 328
- DE-A1-102008 023 751
- DE-U1- 9 315 132

## Beschreibung

Die Erfindung betrifft ein bidirektionales Schwenkgelenk, umfassend einen zylindrischen mit Hohlraum hohlen Außenkörper und einen in dem Hohlraum angeordneten zylindrischen Innenkörper, wobei der Außenkörper und der Innenkörper mit koaxialen Zylinderachsen um eine Gelenkachse relativ zueinander drehbar sind, sowie eine die relative Drehbewegung zwischen dem Außenkörper und dem Innenkörper wahlweise sperrende und freigebende Arretiereinrichtung. Die Erfindung bezieht sich auch auf eine Halte- und Positioniereinrichtung, umfassend in Reihe eine Mehrzahl von miteinander verbundenen Schwenkgelenken und eine Steuereinrichtung zum Freigeben und Blockieren der Schwenkbewegungen der Schwenkgelenke.

Das bidirektionale Schwenkgelenk der genannten Art ist ein arretierbares Drehgelenk mit relativ zueinander und gleichermaßen in beide Drehrichtungen um die Gelenkachse drehbaren, durch Außenkörper und Innenkörper gebildeten Gelenkelementen. Die Arretiereinrichtung des Schwenkgelenks ist eingerichtet, um durch Drehung erreichte relative Drehpositionen von Außenkörper und Innenkörper zu arretieren und freizugeben. Insbesondere handelt es sich bei der Drehung um eine begrenzte, als Schwenkbewegung verstandene Drehung. Der Arretierzustand soll einer möglichst großen Drehmomentenlast zuverlässig widerstehen, der Freigabezustand soll eine optimale Freidrehung gewährleisten, und der Wechsel zwischen Arretier- und Freigabezustand soll besonders einfach und präzise zu bewirken sein. Solche Erfordernisse bestehen insbesondere für Halte- und Positioniereinrichtungen, in denen die Schwenkgelenke hintereinander in Reihe, insbesondere mit sechs Gelenken, miteinander verbunden sind. Derartige Einrichtungen bilden Roboterarme oder Gelenkarme, die insbesondere Bestandteile von medizinischem Gerät zum Beispiel auf chirurgischem Gebiet sind und besonderen Einsatz- und Sicherheitsanforderungen genügen sollen.

Bekannte gattungsgemäße Schwenkgelenke sind hinsichtlich der genannten Anforderungen nicht zufriedenstellend. Es sind Schwenkgelenke mit reibschlüssiger stufenloser Verriegelung (Arretierung) bekannt. Eine solche Verriegelung unterliegt besonderem Verschleiß, und sie kann nur begrenzten Lasten standhalten. Unter hoher, aber auch bei geringer Last, bedingt durch Verschleiß oder Verschmutzung, ist die reibschlüssige Arretierung gegen Durchrutschen anfällig. Man behilft sich mit Schwenkgelenken, die mit gestuften oder stufenlosen Rastverriegelungen ausgestattet sind (DE 102 09 209 A1). Auf Reibung beruhende stufenlose, aber auch mit Rastverriegelung arbeitende bekannte Verriegelungen benötigen zumeist, insbesondere wenn Gelenke vor einer Entriegelung hoher Last ausgesetzt werden, einen durch Hysterese bedingten verhältnismäßig großen Kraftaufwand zum Betätigen. Zudem lassen die bekannten Schwenkgelenke hinsichtlich Steuerung der Verriegelung und Entriegelung zu wünschen übrig. Insbesondere ist ein von Hand oder durch automatisierte Betätigung gleichzeitig ausgelöstes Verriegeln bzw. Entriegeln mehrerer Gelenke, die miteinander verbunden sind, beeinträchtigt. Die bekannten Gelenke sind nicht sterilisierbar und bereits nicht desinfizierbar.

Aus DE 93 15 132 U1 ist ein im Freigabezustand einseitig in Drehrichtung entsperrtes und in Gegenrichtung klemmendes Drehgelenk mit Merkmalen des eingangs genannten Schwenkgelenks bekannt, wobei die Arretiereinrichtung gebildet ist durch einen mit der Gelenkachse konzentrischen Ringraum zwischen dem Außenkörper und dem Innenkörper mit zwischen Ringraumflächen des Ringraums, nämlich zwischen einer Innenfläche des Außenkörpers und einer Außenfläche des Innenkörpers, sich radial verengenden und demgegenüber radial weiteren Umfangsbereichen in abwechselnder Anordnung, wenigstens zwei über den Ringumfang des Ringraums verteilt in diesem angeordnete Klemmorgane, die im Querschnitt jeweils in Umfangsrichtung des Ringraums veränderbar sind, und eine die genannten veränderbaren Querschnitte der Klemmorgane einstellende Betätigungseinrichtung, wobei jedes Klemmorgan zwei Zustände einnimmt, nämlich einen durch einen ersten genannten Querschnitt jedes Klemmorgans bestimmten Haltezustand, in dem jedes Klemmorgan in genannten sich verengenden Umfangsbereichen, die benachbart sind, an der Innenfläche des Außenkörpers und an der Außenfläche des Innenkörpers anliegt, wodurch eine die Klemmorgane zwischen der Innenfläche und der Außenfläche einfangende selbsthaltende Klemmverbindung eingerichtet ist, die in jeder Drehrichtung die relative Drehbewegung zwischen dem Außenkörper und dem Innenkörper sperrt, und einen durch einen zweiten genannten Querschnitt jedes Klemmorgans bestimmten Freigabezustand, in dem die Klemmverbindung zur Freigabe der relativen Drehbewegung aufgehoben ist, und wobei die Betätigungseinrichtung ein Betätigungsmittel umfasst, das zum Einstellen einerseits des Haltezustandes und andererseits des Freigabezustandes auf die Klemmorgane einwirkt.

Der Erfindung liegen die Ziele zugrunde, ein genanntes Rotations-Schwenkgelenk mit stufenloser bidirektionaler Rotationsarretierung zu verwirklichen, dessen Handhabung und Betrieb hinsichtlich zugleich Belastbarkeit der Arretierverbindung, Lebensdauer, Funktionszuverlässigkeit, Sterilisierbarkeit, präziser und weitgehend hysteresefreier Steuerbarkeit der Betriebszustände sowie Robustheit verbessert sein sollen. Auch soll das Schwenkgelenk relativ klein bauend ausführbar sein.

Die Haltezustände der Klemmorgane bewirken das Verriegeln des Schwenkgelenks. In den Freigabezuständen der Klemmorgane ist das Schwenkgelenk gemäß Anspruch 1 erfindungsgemäß entriegelt. Man erreicht erfindungsgemäß eine Reihe von Vorteilen. In ihren Arretierpositionen, nämlich in den Haltezuständen, befinden sich die Klemmorgane mit nicht-deformierbaren, formhaltigen Teilen in spielfreiem Sitz zwischen den Gelenkelementen, nämlich dem Außenkörper und dem Innenkörper. Es sind Formschlussverbindungen hergestellt, die in jeder Drehrichtung relativ zwischen dem Außenkörper und dem Innenkörper relative Drehbewegung durch Klemmung in den radial sich verengenden Umfangsbereichen auch unter besonders großer Momentenlast sperren. Das formschlüssige Einfangen und Klemmen des Klemmorgans beidseitig in der radial verengten, keilartig wirkenden Umfangsgeometrie zwischen dem Außenkörper und dem Innenkörper ist weitgehend frei von Verschleiß der einander berührenden Teile. Man erreicht, dass auch nach längerer Nutzung des Schwenkgelenks ein spielfreier Arretierzustand und infolgedessen Konstanthaltung der Kraftverhältnisse gewährleistet sind. Ein weiterer wesentlicher Vorteil besteht darin, dass der Wechsel zwischen dem Haltezustand und dem Freigabezustand nur einer minimalen Zustandsänderung bedarf, nämlich nur einer kleinsten Querschnittsänderung durch zum Beispiel einfache Längenänderung des Klemmorgans in Umfangsrichtung des konzentrischen Ringraums. Das heißt, dass jedes Klemmorgan in seiner Umfangslängendimension, praktisch entsprechend einer von dem Außenkörper eingeschlossenen Sekantenlinie, nur minimal zu ändern ist, um die Drehbewegung zwischen dem Außenkörper und dem Innenkörper bidirektional freizugeben bzw. zu blockieren. Die genannte Querschnittsänderung gelingt mit dem Betätigungsmittel besonders einfach und gleichfalls zuverlässig. Das Betätigungsmittel muss lediglich derart eingerichtet sein, dass das Klemmorgan in der der genannten Sekante entsprechenden Dimension nur in dem geringen Maß verändert wird, dass das Klemmorgan in Umfangsrichtung des Ringraums beidseitig aus der Formschlussverbindung, nämlich der eingefangenen Position mit nur geringstem Spiel freikommt. Obwohl die Arretierung selbsthaltend eingerichtet ist, ist zum Entriegeln nur eine relativ kleine Kraft erforderlich. Die selbsthaltende Arretierung widersteht einer Drehbelastung in höchstem Maß. Das erfindungsgemäße Schwenkgelenk genügt damit besonderen Sicherheitsanforderungen. Man erreicht eine besonders einfache und zuverlässige Steuerung zum Bewirken des Haltezustandes, des Freigabezustandes sowie des Wechsels zwischen diesen beiden Zuständen mittels des Betätigungsmittels, das entsprechend einfach verwirklich werden kann. Hysterese wird weitgehend ausgeschlossen. Zudem wird eine stufenlose Verriegelung erzielt, die in jeder Position der konzentrisch gelagerten Gelenkelemente übereinstimmende Kraft- und Geometrieverhältnisse gewährleistet, so dass die Funktion des Schwenkgelenks zuverlässig unabhängig von der zur Verriegelung gerade eingenommenen Drehstellung zwischen Außenkörper und Innenkörper bleibt. Aufgrund der zum Wechsel zwischen Haltezustand und Freigabezustand minimal genügenden Geometrieänderung des Klemmorgans in Umfangsrichtung des Ringraums ist auch der Steuerweg zum Wechsel zwischen den beiden Zuständen entsprechend gering. Die Steuerung ist insbesondere hinsichtlich großer Steuergeschwindigkeit, also besonders kurzer Ansprechzeit erheblich vereinfacht. Mittels gemeinsamer Steuereinrichtung lässt sich eine Mehrzahl hintereinander in Reihe geschalteter Schwenkgelenke zuverlässig und präzise zeitgleich betreiben. Das erfindungsgemäße Schwenkgelenk ist, insbesondere im Autoklav, sterilisierbar. Dies erweitert die Einsatzmöglichkeiten. Insbesondere lässt sich das Gelenk für die Knochenchirurgie, die höchste Anforderung an Sterilität stellt, einsetzen.

Eine eingangs genannte Halte- und Positioniereinrichtung zeichnet sich dadurch aus, dass die Schwenkgelenke jeweils durch ein erfindungsgemäßes Schwenkgelenk gebildet sind.

Obgleich das Klemmorgan durch jedes Mittel gebildet sein kann, dessen Querschnitt in Umfangsrichtung des Ringraums, also quer zu sich in Axialrichtung des Schwenkgelenks erstreckenden Radialebenen veränderbar ist, besteht eine besonders bevorzugte praktische Gestaltung darin, dass das Klemmorgan gebildet ist durch zwei in Umfangsrichtung des Ringraums versetzt angeordnete klemmbare Klemmelemente und ein in Umfangsrichtung des Ringraums längenveränderbares Distanzmittel, das die beiden Klemmelemente in Umfangsrichtung des Ringraums voneinander distanziert und relativ zueinander zum Einrichten wenigstens einer der beiden Positionen, nämlich der Halteposition bzw. der Freigabeposition, verlagert. Die Klemmelemente sind nicht deformierbare, formfeste Teile.

Besonders vorteilhaft kann das die beiden Klemmelemente des Klemmorgans verbindende Distanzmittel ein unter Kraft arbeitendes Kraftorgan sein. Die die Klemmelemente beabstandende Distanz unterliegt dann einer Kraft/Weg-Funktion. Zweckmäßig ist das Kraftorgan ein in Umfangsrichtung des Ringraums wirkendes Federmittel. Wenigstens eine Druckfeder wird vorgesehen, wenn das Kraftorgan zum Herstellen des Freigabezustandes gegen Druckkraft zu verkürzen ist. Die Klemmelemente des Klemmorgans können mit mindestens einer Zugfeder verbunden werden, wenn die Distanz der Klemmelemente gegen Zugkraft zu vergrößern ist, um den Freigabezustand zu bewirken. Allgemein ist als Kraftorgan jedes Mittel geeignet, das zum Zusammendrücken oder Auseinanderdrücken des Klemmorgans, also zur entsprechenden Kraftbeaufschlagung der Klemmelemente geeignet ist, nämlich insbesondere statt eines mechanischen Federmittels zum Beispiel ein pneumatisch oder elektrisch wirkendes Mittel. Allgemein ist es von Bedeutung, dass das Distanzmittel bzw. das Kraftorgan lediglich so eingerichtet ist, dass es die Klemmelemente in den Klemmräumen, nämlich den sich verengenden Umfangsbereichen hält.

Eine bevorzugte Gestaltung besteht darin, dass die Klemmelemente des Klemmorgans sich parallel zur Gelenkachse erstreckende Zylinderelemente sind. Durch Wahl der Länge der Zylinderelemente, zum Beispiel in Form von Zylinderstiften, kann die Größe der Haltekraft im Haltezustand besonders eingerichtet werden. Die Länge der Kontaktflächen/-linien der Zylinderelemente mit der Außenkörper-Innenfläche bzw. der Innenkörper-Außenfläche ist ein wesentliches Maß für die Haltekraft, die insbesondere zumindest im Wesentlichen proportional zur Länge der Kontaktflächen eingerichtet werden kann. Es kommen aber auch Ausführungen von Klemmorganen mit Klemmelementen in Betracht, die andere zum Formschluss und Klemmen geeignete Geometrie und Festigkeit aufweisen, insbesondere zum Beispiel Kugeln.

Ein bevorzugte Gestaltung besteht darin, dass der Ringraum in jeweils ein genanntes Klemmorgan aufnehmende Umfangsabschnitte unterteilt ist, wobei jeder Umfangsabschnitt zwei der sich verengenden Umfangsbereiche aufweist, die jeweils einen den Ringraum in seiner Radialdimension verengenden Klemmraum bilden. Diese Gestaltung wird insbesondere in Verbindung mit der Maßnahme vorgesehen, dass jedes Klemmorgan ein Paar Klemmelemente aufweist, die zum Herstellen des Haltezustandes jeweils in die beiden sich verengenden Umfangsbereiche eines genannten Umfangsabschnitts einfassen. Zweckmäßig ist die Außenfläche des Innenkörpers durch Raum-Teilflächen gebildet, die die genannten Umfangsabschnitte des Ringraums bestimmen und zusammen mit der Innenfläche des Außenkörpers die sich verengenden Umfangsbereiche bilden. Zweckmäßig sind die Raum-Teilflächen des Innenkörpers im Drehquerschnitt des Gelenks wenigstens teilweise durch einen Polygonzug bestimmt. In der gleichen Weise kann aber auch die Innenfläche des Außenkörpers als insbesondere polygone Mehreckfläche vorgesehen sein. Stets bildet die Mehreckfläche im Zusammenspiel mit der anderen Ringraumfläche die sich verengenden bzw. die dem gegenüber weiteren Umfangsbereiche. Die genannten Mehreckflächen, vorzugsweise mit geraden oder ebenen Flächen, sind ein allgemeines Merkmal der Erfindung.

Eine Ausgestaltung besteht darin, dass die beiden Klemmelemente eines genannten Klemmorgans jeweils an einer Raum-Teilfläche von zwei benachbarten genannten Raum-Teilflächen einer genannten Ringraumfläche, vorzugsweise an der Außenfläche des Innenkörpers, angeordnet sind. Eine andere Ausgestaltung sieht vor, dass die beiden Klemmelemente eines genannten Klemmorgans an einer gemeinsamen genannten Raum-Teilfläche einer genannten Ringraumfläche, vorzugsweise an der Außenfläche des Innenkörpers, angeordnet sind.

In Verbindung mit den Klemmorganen kann jede Betätigungseinrichtung vorgesehen sein, die sämtliche Klemmorgane verkürzt und/oder verlängert, damit diese jeweils aus dem Haltezustand in den Freigabezustand gelangen und, gemäß einer möglichen Gestaltung, auch umgekehrt in den Haltezustand. Zu diesem Zweck können Mittel vorgesehen werden, die zum Beispiel pneumatisch, elektromechanisch oder mechanisch auf die Klemmorgane einwirken. Eine besonders bevorzugte Maßnahme besteht darin, dass die Betätigungseinrichtung Steuerelemente aufweist, die zum Einstellen des Haltezustandes bzw. des Freigabezustandes an den genannten Klemmelementen angreifen, wobei sie gegen die Kraft des genannten Distanzmittel-Kraftorgans arbeiten.

Insbesondere wird wenigstens ein Steuerelement wenigstens teilweise innerhalb des Ringraums zwischen zwei benachbarten Klemmelementen angeordnet. Vorzugsweise sind in dieser Gestaltung wie auch in den anderen Gestaltungen sämtliche Klemmorgane und/oder Steuerelemente gleich angeordnet und ausgebildet. Die Anordnung des Steuerelements teilweise innerhalb des Ringraums ist zweckmäßig der Art, dass das Steuerelement zum Entriegeln aus einer Nahezu-Berührungsposition mit den Klemmorganen bzw. Klemmelementen in Eingriff mit diesen gelangt. Vorteilhaft wird das Steuerelement mit einem Steuerprofil ausgebildet, das abgeschrägt und/oder gerundet ist, derart, dass eine radiale Steuerbewegung des Steuerelements in den Ringraum hinein, zu dieser Bewegung quer gerichtet, Druckkraft auf vorteilhaft zugleich zwei benachbarte Klemmorgane und/oder Klemmelemente im Ringraum bewirkt. Zum Beispiel wird das Steuerprofil durch einen kugel-, dreieck-, trapezförmigen oder zylindrischen Steuerkopf gebildet. In jedem Fall unterstützen die mit radialer Steuerbewegung arbeitenden Steuerelemente auch eine selbsttätige Justierung der in dem Ringraum angeordneten Elemente.

Eine Gestaltung besteht darin, dass wenigstens einem Umfangsabschnitt des Ringraums, welcher Umfangsabschnitt frei von Distanzelementen der Klemmorgane ist, ein genanntes Steuerelement für zwei benachbarte Klemmelemente zugeordnet ist. Diese Ausgestaltung ist besonders vorteilhaft in Verbindung mit benachbarten Klemmorganen, deren beide Klemmelemente jeweils an einer genannten Teilfläche der Außenfläche des Innenkörpers angeordnet sind.

Eine andere Gestaltung besteht darin, dass wenigstens einem Ringraum-Umfangsabschnitt, in dem das Distanzmittel eines genannten Klemmorgans angeordnet ist, ein genanntes Steuerelement für zwei benachbarte Klemmelemente zugeordnet ist. Diese Gestaltung ist insbesondere für genannte Klemmorgane geeignet, deren beide mit dem Distanzmittel verbundene Klemmelemente an zwei benachbarten Teilflächen der Außenfläche des Innenkörpers angeordnet sind.

Eine noch andere Gestaltung besteht darin, dass wenigstens ein Distanzmittel eines genannten Klemmorgans durch ein genanntes Steuerelement gebildet ist. So kann ein Steuerelement zum Beispiel ein Kraftorgan sein, das die Verlängerung bzw. Verkürzung des Klemmorgans zum Einnehmen des Haltezustandes bzw. des Freigabezustandes bewirkt.

Eine vorteilhafte Maßnahme besteht darin, dass in dem Ringraum wenigstens eine kettenartige Reihe eingerichtet ist, die durch genannte Klemmelemente, genannte Distanzelemente und genannte Steuerelemente gebildet ist, wobei die Kettenglieder, jeweils benachbart, im Freigabezustand der Klemmorgane miteinander in Verbindung stehen. Zum Beispiel ist eine einzige kettenartige Reihe über den Ringumfang geschlossen. Diese geschlossene Reihe wie auch Teilreihen tragen besonders zum Selbstjustieren der Elemente des Schwenkgelenks bei. Beim Entriegeln wird dennoch Interferenz zwischen benachbarten Klemmorganen ausgeschlossen.

Eine besonders einfache und bevorzugte Gestaltung der Betätigungseinrichtung besteht darin, dass sie wenigstens ein Steuerelement aufweist, das zum Einstellen des Haltezustands bzw. des Freigabezustands an zugehörigen genannten Klemmorganen angreift, an dem Innenkörper des Schwenkgelenks gelagert und quer zur Umfangsrichtung des Ringraums stellbewegbar angeordnet ist.

Mehrere, vorzugsweise sämtliche genannte Steuerelemente können mittels eines einzigen Steuerteils stellbewegbar eingerichtet sein, das insbesondere in einem Hohlraum des Innenkörpers des Schwenkgelenks angeordnet und achsparallel zur Zylinderachse des Innenkörpers verstellbar ist. Ein solches Steuerteil kann, zum Beispiel nach Art eines Stößels, mit Steuerflächen ausgebildet sein, die an Teilen, zum Beispiel Steuerstiften, von zugehörigen Steuerelementen anliegen und axiale Bewegung des Steuerteils (Steuerkörpers) in radiale Bewegung der Steuerelemente umsetzen. Besonders vorteilhaft sind am Umfang des Steuerteils angeordnete Steuer-/Anlageflächen, die diagonal gegenüberliegen, so dass sie eine Selbstzentrierung des Steuerteils bewirken. In der Anordnung zwischen den an den Steuerflächen anliegenden Teilen ist das Steuerteil nahezu spielfrei radial gelagert.

Vorteilhaft ist eine den Ringraum begrenzende Ringraumfläche, nämlich die Innenfläche des Außenkörpers oder die Außenfläche des Innenkörpers, eine kreiszylindrische Mantelfläche, und zwar jeweils im Zusammenspiel mit der anderen Ringraumfläche, die mit den genannten Mehreckflächen oder anderen den Ringraum erweiternden/verengenden Flächen ausgebildet ist. Eine bevorzugte Gestaltung besteht darin, dass das Schwenkgelenk mit mehreren genannten Klemmorganen symmetrisch zur Gelenkachse ausgebildet ist. Die symmetrische Anordnung ist zum Zentrieren und Justieren der Gelenkelemente besonders geeignet. Vorteilhaft umfasst das Schwenkgelenk wenigstens sechs der Klemmorgane, die über den Ringumfang des Ringraums gleichmäßig verteilt angeordnet sind. Sechs Klemmorgane, insbesondere in Verbindung mit sechs zugehörigen genannten Mehreckflächen, haben sich zum Optimieren der Größe der Haltekraft als besonders vorteilhaft erwiesen. Aber auch ein erfindungsgemäßes Schwenkgelenk mit zum Beispiel drei gleichen Klemmorganen bzw. zugehörigen Verengungsflächen bietet die genannten Vorteile.

In einer Grundausführung des erfindungsgemäßen Schwenkgelenks kann der Außenkörper ausschließlich über die Klemmorgane an dem Innenkörper radial gelagert sein. Der Ringraum und die Klemmorgane sind dann so eingerichtet und ausgebildet, dass im Freigabezustand zwischen den Klemmorganen und dem Außenkörper ein minimales, gerade ausreichendes Lagerspiel vorhanden ist, um die freie Drehung zuzulassen und den Außenkörper und den Innenkörper in konzentrischer Anordnung aneinander zu lagern. Insbesondere bilden dann Zylinderelemente in Form von Zylinderstiften, nämlich die Klemmelemente der Klemmorgane, Lagerstifte eines Stiftlagers zwischen den beiden Körpern. Grundsätzlich wird allgemein ein minimales Spiel zwischen der Innenfläche des Außenkörpers und den Klemmorganen, insbesondere den Klemmelementen der Klemmorgane, im Freigabezustand an der Außenfläche des Innenkörpers anliegend, so eingerichtet, dass es gerade eben die freie Drehung zulässt.

Zweckmäßig können der Außenkörper und der Innenkörper über wenigstens ein außerhalb des Ringraums angeordnetes Radiallager schwenk-/drehbeweglich aneinander gelagert sein. Die Klemmorgane bleiben dann frei von der genannten Lagerfunktion.

Eine besondere Gestaltung der erfindungsgemäßen Halte- und Positioniereinrichtung besteht darin, dass die Steuereinrichtung entsprechend der Anzahl der in Reihe miteinander verbundenen Schwenkgelenke Steuerteile umfasst, die jeweils durch einen wenigstens eine Steuerkontur aufweisenden Steuerkörper gebildet sind, wobei jeder zu einem Schwenkgelenk zugehörige, in Richtung der zugehörigen Gelenkachse axial stellbewegbare Steuerkörper wenigstens eine unter flachem Verlauf zu der Gelenkachse ausgerichtete Steuerfläche aufweist, die entsprechend der axialen Stellposition des Steuerkörpers wenigstens ein zugehöriges, quer zur Gelenkachse bewegbares Steuerelement des zugehörigen Schwenkgelenks zum Freigeben bzw. Blockieren der Schwenkbewegung steuert.

Zwischen dem Außenkörper und dem Innenkörper des Schwenkgelenks kann ein Haltemittel angeordnet sein, das sämtliche Klemmorgane mit zum Innenkörper gerichteten Haltekräften beaufschlagt. Ein solches Haltemittel wird vorteilhaft durch ein elastisches Haltemittel, zweckmäßig durch eine sogenannte Wurmfeder gebildet, die an sämtliche Klemmorgane, über den vollen Umfang des Ringraums geschlossen, angelegt wird. Zum Beispiel wird das Haltemittel an die genannten Klemmelemente der Klemmorgane angelegt, so dass sämtliche Klemmelemente ungestört durch die genannte Querschnittsänderung der Klemmorgane in gesicherter Anlage an der Außenfläche des Innenkörpers bleiben.

Auf die genannten und noch andere zweckmäßige und vorteilhafte Ausgestaltungen der Erfindung sind Unteransprüche gerichtet. Lediglich besonders zweckmäßige und vorteilhafte Ausbildungsformen und -möglichkeiten werden anhand der folgenden Beschreibung der in der schematischen Zeichnung dargestellten Ausführungsbeispiele näher beschrieben. Jede beschriebene Einzel- oder Detailgestaltung innerhalb eines Ausführungsbeispiels ist als unabhängiges Detailbeispiel für andere nicht oder nicht vollständig beschriebene, unter die Erfindung fallende Ausführungen und Gestaltungen zu verstehen. Insbesondere wird als allgemeines Merkmal ein solches Merkmal bezeichnet und verstanden, das auch isoliert von anderen Merkmalen einer Kombination oder eines Ausführungsbeispiels zu dem erfindungsgemäßen Erfolg der allgemeinen Lehre der Erfindung insbesondere nach einem Hauptanspruch beiträgt. Es zeigen
- Fig. 1 und 2: in Stirnansicht erfindungsgemäße Schwenkgelenke einer ersten Bauart,
- Fig. 3A bis 3C: axonometrische Ansichten eines erfindungsgemäßen Schwenkgelenks einer zweiten Bauart mit Schnittansicht gemäß Fig. 3A und Stirnansichten gemäß Fig. 3B und 3C,
- Fig. 3D: im Längsschnitt eine Schwenkeinrichtung der Art gemäß Fig. 3A bis 3C und
- Fig. 4: eine erfindungsgemäße Halte- und Positioniereinrichtung mit erfindungsgemäßen Schwenkgelenken.

In sämtlichen Figuren sind, ungeachtet von Ausführungsänderungen, für gleiche Teile, die erfindungsgemäß gleichwirkend und gleichwertig sind, die gleichen Bezugszeichen verwendet worden.

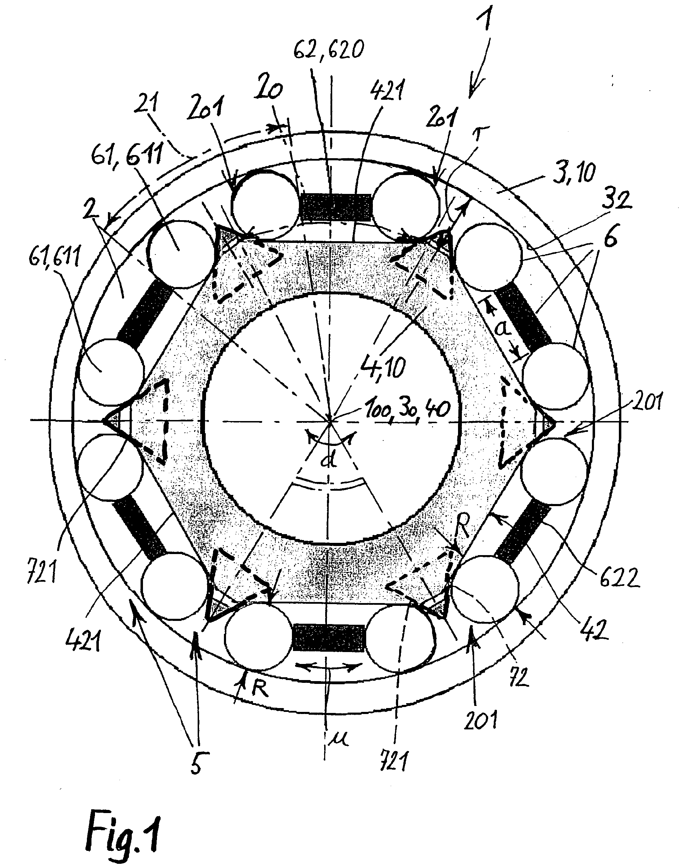

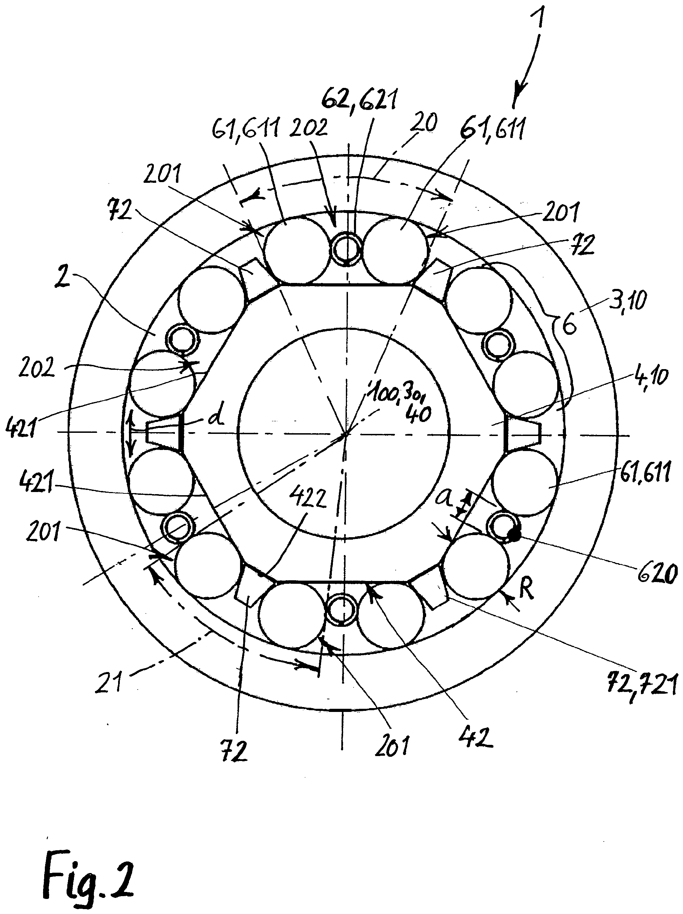

Fig. 1 und 2 zeigen erfindungsgemäße Schwenkgelenke 1, die jeweils in konzentrischer Anordnung einen Außenkörper 3 und einen Innenkörper 4, einen zwischen den beiden Körpern 3, 4 gebildeten Ringraum 2 sowie darin angeordnete Klemmorgane 6 umfassen. Der Außenkörper 3 und der Innenkörper 4 sind jeweils durch einen kreiszylindrischen Hohlkörper gebildet, wobei die Zylinderachsen 30, 40 koaxial in einer Gelenkachse 100 des Schwenkgelenks 1 zusammenfallen. Der Außenkörper 3 und der Innenkörper 4 sind Gelenkelemente 10, die um die Gelenkachse 100 relativ zueinander in bidirektionaler Richtung d drehbar sind.

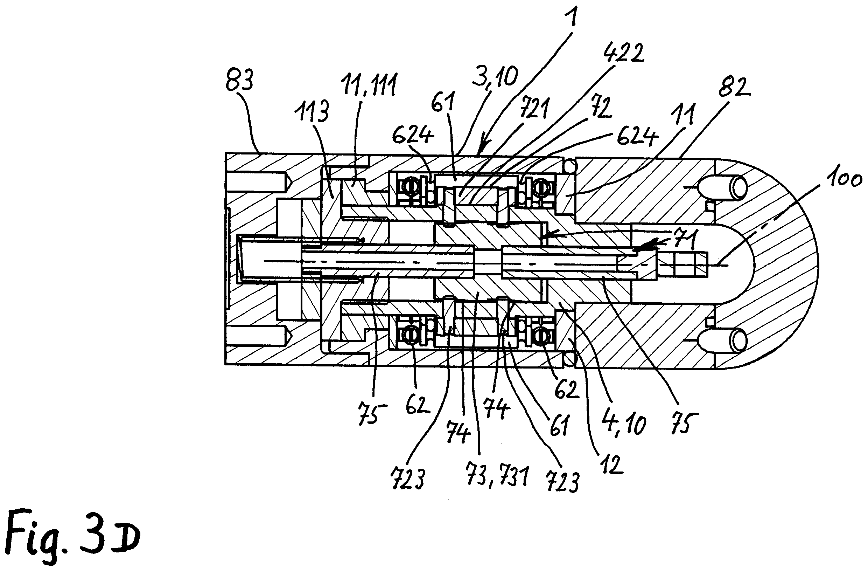

Es sind sechs gleiche Klemmorgane 6 vorgesehen, die über den Umfang des Ringraums 2, in dessen Umfangsrichtung u versetzt, gleichmäßig verteilt sind und ein Radiallager zur Lagerung der beiden Körper 3, 4 aneinander bilden können. Die Schwenkgelenke 1 der Fig. 1, 2 sollen aber bevorzugt mit Radiallagern ausgestattet sein, die außerhalb des Ringraums 2 in in Fig. 1, 2 nicht dargestellten Stirnbereichen angeordnet sind. Es werden als allgemeines Merkmal Radiallager vorgesehen, wie sie zum Beispiel entsprechend Fig. 3D ausgeführt sein können.

Der Ringraum 2 erstreckt sich in Richtung der Schwenkachse 100 mit einer bestimmten, den Zylinderlängen der beiden Körper 3, 4 entsprechenden Ringraumlänge. In der Radialdimension ist der Ringraum 2 mit Anpassung auf die Klemmorgane 6 besonders gestaltet.

Der Außenkörper 3 weist eine Zylinder-Innenfläche (kreiszylindrische Innenfläche) 32 auf, die im Zylinderquerschnitt kreisförmig ist. Hingegen ist die Zylinder-Außenfläche 42 des Innenkörpers 4 durch sechs gleiche ebene Raum-Teilflächen 421 gebildet, wobei im Zylinderquerschnitt eine Polygonzug mit sechs gleichen, den Raum-Teilflächen 421 entsprechenden geraden Polygonabschnitten zu sehen ist. Zwischen den Polygonabschnitten befinden sich gleiche, im Verhältnis zu der genannten Polygonabschnittslänge kurzstückige Flächenzwischenabschnitte 422. Man erkennt, dass die Raum-Teilflächen 421 im Verhältnis zu der Innenfläche 32 des Außenkörpers 3 Sekantenflächen bilden, die über den Ringumfang des Ringraums 2 sich abwechselnd radial verengende und erweiternde Umfangsbereiche 201, 202 bilden. So gehören zu jeder Raum-Teilfläche 421 im Paar die engen bzw. sich verengenden Umfangsbereiche 201 und dazwischen der erweiterte bzw. sich erweiternde Umfangsbereich 202.

Jedes Klemmorgan 6 ist zugehörig an einer genannten Raum-Teilfläche 421 angeordnet. Es umfasst zwei den engen Umfangsbereichen 201 zugeordnete formfeste Klemmelemente 61 und ein mit diesen verbundenes Distanzmittel 62, das die beiden Klemmelemente 61 im Umfangsbereich 202 mit Distanz a beabstandet. Die Distanz a unterliegt einer durch das Distanzmittel 62 bestimmten Kraft/Weg-Funktion. Das Distanzmittel 62 ist ein Kraftorgan 620, nämlich im Beispiel der Fig. 1 eine Druckfeder 621. Unter einer solchen Druckfeder 621 können auch mehrere parallel angeordnete Druckfedern verstanden werden. Zum Beispiel ist jeweils eine Druckfeder an axialen Längsenden eines Klemmelementepaars angeordnet. Die Klemmelemente 61 sind stiftartige Zylinderelemente 611 mit Kreisquerschnitt, die sich parallel mit der Gelenkachse 100 erstrecken. Die Klemmelemente 61 weisen einen Kreisdurchmesser R auf, der größer ist als die kleinste radiale Dimension der verengenden Umfangsbereiche 201, jedoch kleiner als die radiale Weite des zugehörigen Umfangsbereichs 202, der sich zwischen den sich verengenden Umfangsbereichen 201 erweitert. Klemmelemente mit einem solchen größeren Durchmesser eines Elements mit Kreisquerschnitt oder allgemein mit größerem Durchmesser als der Verengungsdurchmesser sind ein allgemeines Merkmal.

Das Klemmorgan 6 ist derart eingerichtet, dass es unter Wirkung des Distanzmittels 62 zwei Zustände einnimmt, nämlich einen Haltezustand und einen Freigabezustand. Der Haltezustand ist dadurch bestimmt, dass die beiden Klemmelemente 61 des Klemmorgans 6 zugehörig in die sich verengenden Umfangsbereiche 201 einfassen. Dabei liegt das Klemmelement 61 formschlüssig an der Außenkörper-Innenfläche 32 sowie an der zugehörigen Raum-Teilfläche 421 des Innenkörpers 42 an, und zwar in tangentialer Berührung. Der Haltezustand ist dadurch bewirkt und wird dadurch aufrechterhalten, dass das Distanzmittel 62 die Klemmelemente 61 mit größter Distanz a auseinander drängt, so dass die Klemmelemente 61 in den engen Umfangsbereichen 201 gefangen sind. Es resultiert, dass der Außenkörper 3 und der Innenkörper 4 in dem beschriebenen Haltezustand, der von sämtlichen Klemmorganen 6 zeitgleich eingenommen wird, drehfest aneinander arretiert und so gegen jede relative Drehbewegung gesperrt sind. Der Freigabezustand entsteht im Ausführungsbeispiel der Fig. 1, 2 durch ein Verkleinern der Distanz a des Distanzmittels 62. Wie dies bewirkt wird, wird nachfolgend noch beschrieben.

Man erkennt, dass das Klemmorgan 6 nach Maßgabe der Distanz a entsprechend der Richtung u des Umfangs des Ringraums 2 im Querschnitt veränderbar ist. Im Haltezustand ist dieser Querschnitt maximal; und der Freigabezustand wird bereits durch eine minimale Verkleinerung des Querschnitts bewirkt, und zwar durch minimale Reduktion der Distanz a, wobei beide Klemmelemente 61 aus den beiden Umfangsbereichen 201 zugleich und vorzugsweise in gleichem Maß verdrängt werden. Insoweit besteht ein allgemeines Merkmal darin, dass sich der Umfangsbereich 201, entsprechend seiner Verengung größer werdend, in den Umfangsbereich 202 öffnet. Infolgedessen kommen die Klemmelemente 61 beim Entriegeln progressiv frei. Im Ausführungsbeispiel ist die Geometrie der sich verengenden Umfangsbereiche 201 bzw. des entsprechend erweiternden Übergangs in den Umfangsbereich 202 durch die sekantenartige Lage der Raum-Teilflächen 421 zu der Kreisform der anderen Ringraumfläche erreicht. Mit dieser ein allgemeines Merkmal bildenden Geometrie wirkt der Kreisquerschnitt der Zylinderelemente 611 zusammen, die im Haltezustand nur tangential an der einen Raum-Teilfläche und der kreisförmigen Kontur der anderen Ringraumfläche anliegt.

Die Raum-Teilflächen 421 bestimmen Umfangsabschnitte 20 des Ringraums 2. Benachbarte Umfangsabschnitte 20 sind jeweils durch einen Umfangsabschnitt 21 voneinander getrennt, der den Flächenzwischenabschnitt 422 einschließt und frei von Distanzmitteln 62 der Klemmorgane 6 ist. So befinden sich die Umfangsabschnitte 21 zwischen den Klemmorganen 6.

In jeden Umfangsabschnitt 21 fasst ein radial geführtes Steuerelement 72 ein mit einem in Fig. 1 im Drehquerschnitt des Gelenks dreieckförmigen Steuerkopf 721 und mit einem in Fig. 2 trapezförmigen Steuerkopf 721. Die jeweils gleich eingerichteten Steuerelemente 72 sind mittels nicht dargestellter Radialführungen an dem Innenkörper 4 gelagert und zum Verriegeln bzw. Entriegeln des Schwenkgelenks 1 in Richtung r hin und her bewegbar. In Fig. 1 und 2 soll jeweils der Verriegelungszustand des Schwenkgelenks 1 mit den Haltezuständen sämtlicher Klemmorgane 6 dargestellt sein. Die Steuerelemente 72 befinden sich in entsprechenden übereinstimmenden Positionen, in denen sie den Form-/Klemmsitz der Klemmelemente 61 in den Umfangsbereichen 201 unbeeinträchtigt zulassen, wobei sie sich in einer Nahezu- oder Quasiberührung, wie in Fig. 1 und 2 dargestellt, mit den Klemmelementen 61 befinden. Eine solche Quasi-Berührung ist ein bevorzugtes allgemeines Merkmal der Erfindung. Es sind damit definierte Innenpositionen, nämlich Ausgangspositionen der Steuerelemente 72 zum Entriegeln eingerichtet. Vorteilhaft erstreckt sich der Steuerkopf 721 des Steuerelements 72 wenigstens nahezu über die axiale Länge der zugehörigen Klemmelemente 61. Dies ist ein allgemeines Merkmal.

Gemäß den Ausführungsbeispielen der Fig. 1 und 2 ist jedes Steuerelement 72 während der Haltezustände der Klemmorgane 6 wenigstens teilweise innerhalb des Ringraums 2 zwischen zwei benachbarten Klemmelementen 61 bzw. Klemmorganen 61 angeordnet. Wie in Fig. 1 und 2 nicht näher dargestellt, lassen sich die Steuerelemente 72 aus der genannten Ausgangsposition heraus einheitlich und gleichermaßen radial nach außen weiter in den Ringraum 2 hinein, nämlich in Außenpositionen bewegen, und zwar mit einer in Fig. 1, 2 nicht dargestellten Betätigungseinrichtung, die die Steuerelemente 72 einheitlich in den Innen- bzw. Außenpositionen hält. Dies betrifft ein allgemeines Merkmal.

Man erkennt, dass die Klemmelemente 61 mittels der in die Außenposition gesetzten Steuerelemente 72 jeweils gegen die Druckkraft des Kraftorgans 620 aus der Klemmposition zum Bewirken des Freigabezustandes frei kommen. Die radiale Steuerbewegung kann aufgrund der bereits beschriebenen geometrischen Verhältnisse minimal bleiben, und sie wird durch die beschriebenen Formen des Steuerkopfes 721 unterstützt. Die bzw. jede Anordnung, mit der jedes Steuerelement zugleich auf zwei Klemmorgane einwirkt, ist ein allgemeines Merkmal. Es wird eine Entkopplung benachbarter Klemmorgane 6 erreicht, indem Bewegungen/Querschnittsänderungen der Klemmorgane 6 durch die Steuerelemente 72 dominiert werden. Rückstellkräfte benachbarter Klemmorgane 6 sind frei von Interferenz.

Man erkennt weiterhin, dass die Klemmorgane 6 und die Steuerelemente 72 während der Freigabezustände über eine geschlossene Ringanordnung, nämlich eine geschlossene Reihe, umfassend die Klemmelemente 61, die Distanzmittel 62 sowie die Steuerelemente 72, durch Berührung miteinander in Verbindung stehen.

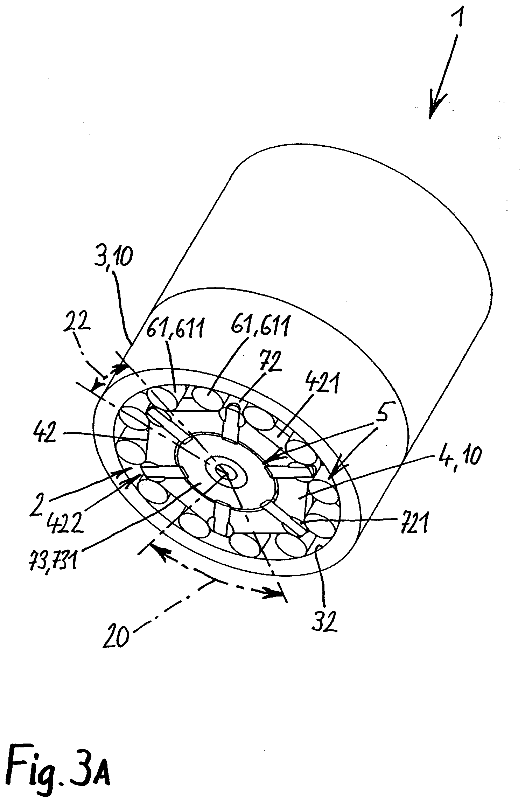

Ein im Folgenden beschriebenes erfindungsgemäßes Schwenkgelenk 1 gemäß Fig. 3A bis 3D umfasst, wie die zuvor beschriebenen Schwenkgelenke, in gleicher Weise einen Außenkörper 3, einen Innenkörper 4 mit sechs Raum-Teilflächen 421, einen entsprechend mit Umfangsbereichen 201 und 202 unterteilten Ringraum 2 und darin angeordnete Klemmorgane 6. Nachfolgend werden nur Unterschiede und weitere Details beschrieben.

Der Ringraum 2 ist in sechs Umfangsabschnitte 20 unterteilt, denen jeweils ein Klemmorgan 6 mit zwei Klemmelementen 61 und einem Distanzmittel 62 zugeordnet ist. Die beiden Klemmelemente 61 eines Klemmorgans 6 sind jeweils an einer genannten Raum-Teilfläche von zwei benachbarten Raum-Teilflächen 421 angeordnet. Das Distanzmittel 62 des Klemmorgans 6 ist jeweils in einem Umfangsabschnitt 22 angeordnet. So erstreckt sich jedes Klemmorgan 6 in Umfangsrichtung u des Ringraums 2 über einen Eckbereich, nämlich einen Flächenzwischenabschnitt 422 zwischen benachbarten Raum-Teilflächen 421. Der Umfangsabschnitt 22 entspricht diesem Eckbereich.

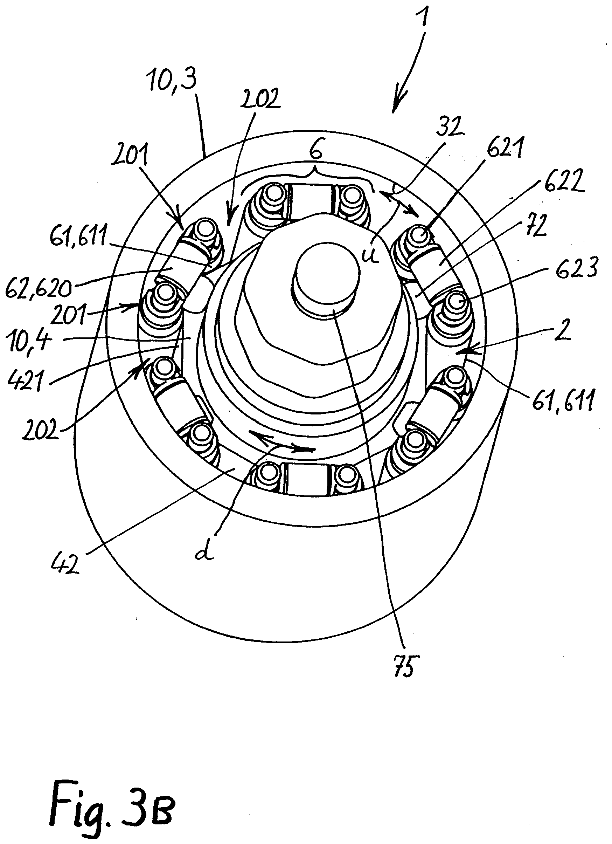

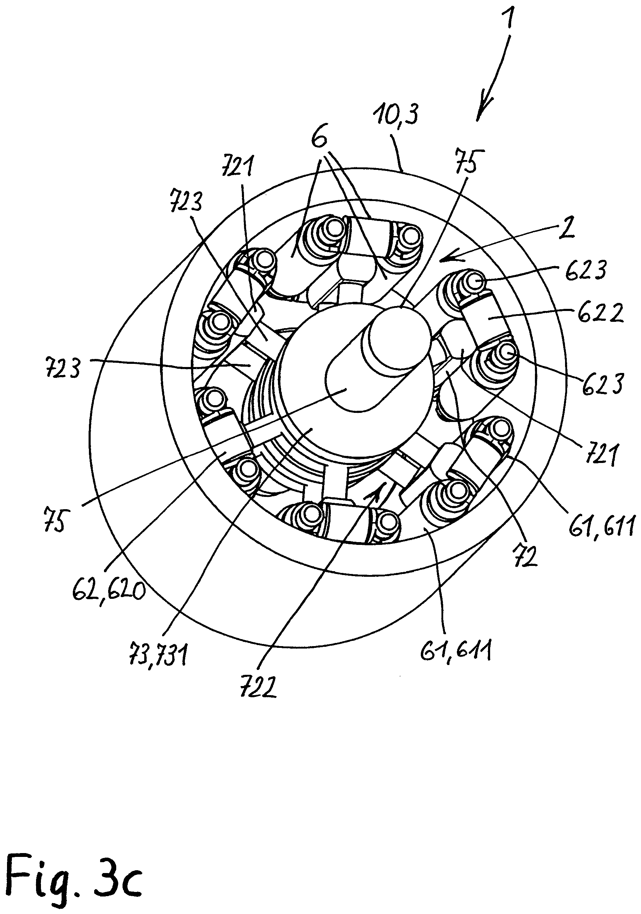

Das Distanzmittel 62 ist ein Zugkraft aufbringendes Kraftorgan 620, zum Beispiel, wie in Fig. 3B und 3C, ein Paar von Zugfedern 622, die stirnseitige Längsenden der beiden Klemmelemente 61 des Klemmorgans 6 miteinander verbinden. Wie aus Fig. 3C und 3B ersichtlich, weisen Zylinderelemente 611 Axialstifte 623 auf, an denen die Zugfedern 622 mit Federösen festgesetzt sind.

Gleich eingerichtete Steuerelemente 72 sind jeweils dem Umfangsabschnitt 22 des Ringraums 2 zugeordnet. Die Steuerelemente 72 weisen einen im Wirkungsquerschnitt kreisförmigen zylindrischen Steuerkopf 721 und einen Steuerfuß 722 mit zwei Steuerstiften 723 auf. Mittels der Steuerstifte 723 ist das Steuerelement 72 an dem Innenkörper 4 radial beweglich geführt. Die axiale Länge des Steuerkopfes 721 entspricht der Länge der zugehörigen Klemmelemente 61.

Fig. 3A und 3B zeigen das Schwenkgelenk 1 im Verriegelungszustand. Man erkennt, auch anhand der Fig. 3D, dass die Haltezustände der Klemmorgane 6 dadurch bewirkt sind, dass sich die Steuerköpfe 721 der Steuerelemente 72 gerade nicht in Eingriff bzw. Berührung mit den Klemmelementen 61 befinden, so dass die Klemmelemente 61 jedes Klemmorgans 6 mittels des Distanzmittels 62 unter Zugkraft in die sich verengenden Umfangsbereiche 201 gepresst werden. Es resultiert der bereits beschriebene Form-/ Klemmschluss mit tangentialer Berührung.

Fig. 3C stellt das entriegelte Schwenkgelenk 1 mit den Freigabezuständen der Schwenkorgane 6 dar. Zum Verdeutlichen der Darstellung ist das Schwenkgelenk 1 in Fig. 3C von dem Innenkörper 4 befreit. Im Unterschied zu Fig. 3B sind die Steuerelemente 72 aus ihren gleichen Innenpositionen radial nach außen in die gleichen Außenpositionen gesteuert. Jedes Steuerelement 72 fasst mit gerundeter Fläche des Steuerkopfes 721 zwischen die zugehörigen beiden Klemmelemente 61, so dass diese gegen die Zugkraft der Zugfedern 622 in Umfangsrichtung u des Ringraums 2 verdrängt werden und infolgedessen aus den Umfangsbereichen 201 freikommen.

Anhand der Fig. 3C und 3D ist eine Betätigungseinrichtung 7 mit einem Betätigungsmittel 71 dargestellt, das zum Einstellen einerseits sämtlicher Haltezustände und andererseits der Freigabezustände auf die Klemmorgane 6 einwirkt.

Das Betätigungsmittel 71 umfasst ein Steuerteil 73 sowie Steuerstangen 75. Das Steuerteil 73 ist in dem Hohlraum des Innenkörpers 4 axial verschieblich angeordnet. Das Steuerteil 73 ist als kreiszylinderförmiger Steuerkörper 731 ausgeführt, der mit zwei Steuerstangen 75, die sich in der Gelenkachse 100 erstrecken, fest verbunden ist. Die Axialführung des Steuerteils 73 kann mittels radialer Lagerung der Steuerstangen 75 an beiden Stirnenden des Schwenkgelenks 1 ausgebildet sein.

Wie aus Fig. 3C und 3D ersichtlich, ist der Steuerkörper 731 an seiner Zylinderfläche mit Steuerflächen 74 ausgestattet, die den Steuerelementen 72 zugeordnet sind. Die Steuerflächen 74 verlaufen flach zur Gelenkachse 100, und zwar im Ausführungsbeispiel mit gleichen Flächen, nämlich mit geraden flachen Schrägflächen. Jeweils ein Paar Schrägflächen ist den beiden Steuerstiften 723 eines Steuerelements 72 zugeordnet. Im Übrigen sind die Steuerflächen 74 am Umfang des Steuerteils 73 paarweise diagonal einander gegenüberliegend angeordnet.

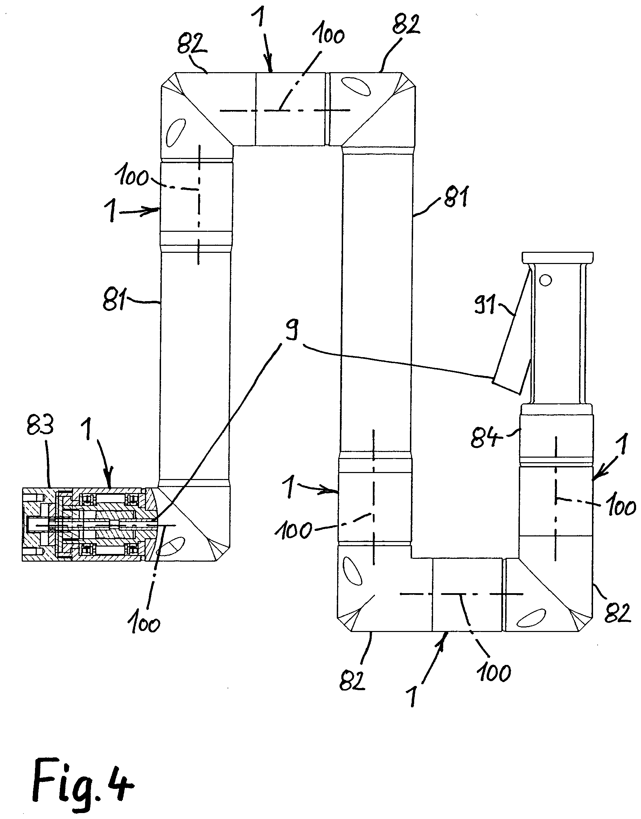

Das in Fig. 3D dargestellte Schwenkgelenk 1 ist dort Bestandteil einer Halte- und Positioniereinrichtung 8, wie sie in Fig. 4 im Beispiel dargestellt ist. Die Halte- und Positioniereinrichtung 8 weist an einem Befestigungsende ein Befestigungsstück 83 sowie an einem Arbeitsende ein Anschlussstück 84 auf. In Fig. 3D ist das Befestigungsende dargestellt. Das Schwenkgelenk 1 gemäß Fig. 3D ist zwischen das Befestigungsstück 83 und ein Eckstück 82 gesetzt. Die Verbindung mit dem Befestigungsstück 83 und dem Eckstück 82 ist über Radiallager 11 hergestellt. Ein endseitiges Radiallager 111, befestigt mittels einer Stirnmutter 113, bildet zugleich ein Axiallager, und eckseitig ist ein ein Axiallager 12 bildender Lagerring vorgesehenen.

Wie aus Fig. 3D ersichtlich, sind die Axialstifte 623 der Klemmelemente 61 jeweils mit einer Taille 624 ausgebildet, die sich zwischen dem Zylinderelement 611 und der Befestigungsstelle für die Zugfeder 622 befindet. Die Taillen 624 nehmen ein Haltemittel auf, nämlich ein um sämtliche Klemmorgane 6 gelegtes elastisches Spannmittel in Form von zum Beispiel ringförmig geschlossenen Wurmfedern, die jeweils am Ende des Schwenkgelenks 1 über die Taillen 624 gelegt sind. Die beiden Wurmfedern sind in Fig. 3D sowie auch in der übrigen Zeichnung nicht dargestellt.

Gemäß Fig. 4 umfasst die Halte- und Positioniereinrichtung 8 sechs erfindungsgemäße Schwenkgelenke 1.

Wie an sich bekannt, sind die Schwenkgelenke 1 über rohrförmige 90°-Eckstücke 82 und Rohr-Arme 81 aneinandergeschlossen. Die Einrichtung 8 ist am Arbeitsende zum Beispiel mit dem Griff- und Anschlussstück 84 ausgestattet, das zum Anschluss an ein anzuschließendes, nicht dargestelltes Gerät od. dgl. eingerichtet ist. Die Fig. 4 zeigt die Einrichtung 8 in unverschwenktem Zustand. Das heißt, dass die Gelenke und sämtliche Arme und Stücke mit ihren Achsen in einer Ebene (Blattebene) liegen.

Die Einrichtung 8 ist mit einer Steuereinrichtung 9 ausgestattet. Eine solche Steuereinrichtung umfasst, wie bekannt, ein Gestänge mit Steuerstangen 75, die sich in den Schwenkgelenken 1 und den Rohr-Armen 81 erstrecken und in den Eckstücken 82 über nicht dargestellte Wippelemente miteinander verbunden sind, um axiale Bewegungen der Stangen 75 mit einem Winkel von 90° zu übertragen bzw. umzulenken.

Eine Besonderheit der erfindungsgemäßen Halte- und Positioniereinrichtung 8 besteht darin, dass die erfindungsgemäßen Schwenkgelenke 1 die mit ihnen erreichten Vorteile zum Handhaben und Betreiben der Einrichtung 8 zur Geltung bringen und die Steuereinrichtung 9 wesentlich verbessert ist. So umfasst die Steuereinrichtung 9 entsprechend der Anzahl der in Reihe miteinander verbundenen Schwenkgelenke 1 die Steuerteile 73. Ein solches Steuerteil 73 in jedem Schwenkgelenk 1 zeichnet sich allgemein dadurch aus, dass es eine mit der Gelenkachse 10 zusammenfallende Axialbewegung in radiale Steuerbewegungen der Steuerelemente 72 umsetzt. Die Steuerteile sind, wie zu Fig. 3D beschrieben, einfache Steuerkörper 731, die die unter flachem Verlauf zu der Gelenkachse 100 ausgerichteten Steuerflächen 74 aufweisen, die jeweils einfach oder mehrfach einem Steuerelement zugeordnet sind. Im Ausführungsbeispiel der Fig. 3D sind jedem Steuerelement 72 zwei gleichwirkende Steuerflächen 74 zugeordnet, jeweils nämlich dem Steuerstift 723 des Steuerelements 72.

Wie allgemein und anhand der Ausführungsbeispiele beschrieben, kann der Steuerweg der Steuerelemente 72 auf ein minimales Maß eingerichtet werden, und zwar entsprechend einem besonders flachen Verlauf einer insbesondere geraden Schräge der Steuerfläche 74. Zweckmäßig endet die flache Steuerfläche 74 endseitig jeweils in einem zur Gelenkachse parallelen geraden Flächenabschnitt. Dieser kann axial so klein ausgebildet sein, insbesondere in Anpassung an die Geometrie der Steuerfüße 722, dass die Steuerelemente 72 gesichert in ihren Endlagen (Innenpositionen/Außenpositionen) sitzen und so der axiale Ansprechweg auf ein Minimum reduziert ist.

Wie üblich arbeitet das Gestänge an dem Befestigungsende der Einrichtung 8 gegen ein in Fig. 3D, 4 nicht dargestelltes Rückstellorgan, zum Beispiel gegen eine an einer nicht dargestellten Befestigungsfläche festgelegte Druckfedereinrichtung. Das Befestigungsstück 83 ist zum Anschluss, zum Beispiel durch Schraubverbindung, an die Befestigungsfläche eingerichtet. Das Griff-/Anschlussstück 84 ist mit einem bewegbaren Betätigungselement 91 versehen, das, wie an sich bekannt, zum axialen Verstellen der Steuerstangen 75 der Steuereinrichtung 9 eingerichtet ist.

Wie aus Fig. 3D in Verbindung mit Fig. 4 ersichtlich, lassen sich die Steuerstangen 75 der Steuereinrichtung 9 mechanisch einfach mit dem Steuerteil 73 verbinden, und zwar zum Beispiel dadurch, dass die axialen Enden des Steuerteils 73 jeweils mit der Steuerstange 75 fest verbunden sind. Zweckmäßig kann diese Verbindung, die gegebenenfalls nur einseitig vorgesehen ist, eine Justierverbindung sein. Dieses allgemeine Merkmal der Erfindung kann zum Beispiel durch wenigstens eine Gewindeverbindung verwirklicht sein.

## Patentansprüche

1. Bidirektionales Schwenkgelenk (1), umfassend einen zylindrischen mit Hohlraum (31) hohlen Außenkörper (3) und einen in dem Hohlraum (31) angeordneten zylindrischen Innenkörper (4), wobei der Außenkörper (3) und der Innenkörper (4) mit koaxialen Zylinderachsen (30,40) um eine Gelenkachse (100) relativ zueinander drehbar sind, sowie eine die relative Drehbewegung zwischen dem Außenkörper (3) und dem Innenkörper (4) wahlweise sperrende und freigebende Arretiereinrichtung (5), wobei die Arretiereinrichtung (5) gebildet ist durch einen mit der Gelenkachse (100) konzentrischen Ringraum (2) zwischen dem Außenkörper (3) und dem Innenkörper (4) mit zwischen Ringraumflächen des Ringraums (2), nämlich zwischen einer Innenfläche (32) des Außenkörpers (3) und einer Außenfläche (42) des Innenkörpers (4), sich radial verengenden und demgegenüber radial weiteren Umfangsbereichen (201, 202) in abwechselnder Anordnung, wenigstens zwei über den Ringumfang des Ringraums (2) verteilt in diesem angeordnete Klemmorgane (6), die im Querschnitt jeweils in Umfangsrichtung (u) des Ringraums (2) veränderbar sind, und eine die genannten veränderbaren Querschnitte der Klemmorgane (6) einstellende Betätigungseinrichtung (7), wobei jedes Klemmorgan (6) zwei Zustände einnimmt, nämlich einen durch einen ersten genannten Querschnitt jedes Klemmorgans (6) bestimmten Haltezustand, in dem jedes Klemmorgan (6) in genannten sich verengenden Umfangsbereichen (201), die benachbart sind, in Formschlussverbindung an der Innenfläche (32) des Außenkörpers (3) und an der Außenfläche (42) des Innenkörpers (4) anliegt, wodurch eine die Klemmorgane (6) zwischen der Innenfläche (32) und der Außenfläche (42) einfangende selbsthaltende Klemmverbindung eingerichtet ist, die in jeder Drehrichtung (d) die relative Drehbewegung zwischen dem Außenkörper (3) und dem Innenkörper (4) sperrt, und einen durch einen zweiten genannten Querschnitt jedes Klemmorgans (6) bestimmten Freigabezustand, in dem die Klemmverbindung zur Freigabe relativer Drehbewegung aufgehoben ist, und wobei die Betätigungseinrichtung (7) ein Betätigungsmittel (71) umfasst, das zum Einstellen einerseits des Haltezustandes und andererseits des Freigabezustandes auf die Klemmorgane (6) einwirkt, **dadurch gekennzeichnet, dass** das Betätigungsmittel (71) zum Einstellen des Freigabezustandes durch Längenänderung des Klemmorgans (6) in Umfangsrichtung (u) des Ringraums (2) derart eingerichtet ist, dass die Klemmorgane (6) in Umfangsrichtung (u) des Ringraums (2) beidseitig aus der Formschlussverbindung freikommen, wodurch die Drehbewegung zwischen dem Außenkörper (3) und dem Innenkörper (4) bidirektional freigegeben wird.

2. Schwenkgelenk nach Anspruch 1, **dadurch gekennzeichnet, dass** wenigstens ein genanntes Klemmorgan (6) gebildet ist durch zwei in Umfangsrichtung (u) des Ringraums (2) versetzt angeordnete klemmbare Klemmelemente (61) und ein in Umfangsrichtung (u) des Ringraums (2) längenveränderbares Distanzmittel (62), das die beiden Klemmelemente (61) in Umfangsrichtung (u) des Ringraumes (2) voneinander distanziert und relativ zueinander zum Einrichten wenigstens einer der beiden Positionen, nämlich der Halteposition bzw. der Freigabeposition, verlagert.

3. Schwenkgelenk nach Anspruch 1 oder 2, **dadurch gekennzeichnet, dass** der Ringraum (2) in jeweils ein genanntes Klemmorgan (6) aufnehmende Umfangsabschnitte (20) unterteilt ist, wobei jeder Umfangsabschnitt (20) zwei der sich verengenden Umfangsbereiche (201) aufweist.

4. Schwenkgelenk nach Anspruch 3, **dadurch gekennzeichnet, dass** jedes Klemmorgan (6) ein Paar Klemmelemente (61) aufweist, die zum Herstellen des Haltezustandes jeweils in die beiden sich verengenden Umfangsbereiche (201) eines genannten Umfangsabschnitts (20) einfassen.

5. Schwenkgelenk nach Anspruch 4, **dadurch gekennzeichnet, dass** die eine Ringraumfläche des Ringraums (2), nämlich die Außenfläche (42) des Innenkörpers (4) oder die Innenfläche (32) des Außenkörpers (3), durch Raum-Teilflächen (421) gebildet ist, die die genannten Umfangsabschnitte (20) des Ringraums (2) bestimmen und zusammen mit der anderen Ringraumfläche des Ringraums (2) die sich verengenden Umfangsbereiche (201) bilden.

6. Schwenkgelenk nach Anspruch 5, **dadurch gekennzeichnet, dass** die beiden Klemmelemente (61) eines Klemmorgans (6) jeweils an einer Raum-Teilfläche von zwei benachbarten genannten Raum-Teilflächen (421) angeordnet sind.

7. Schwenkgelenk nach Anspruch 5 oder 6, **dadurch gekennzeichnet, dass** die beiden Klemmelemente (61) eines Klemmorgans (6) an einer gemeinsamen genannten Raum-Teilfläche (421) angeordnet sind.

8. Schwenkgelenk nach einem der Ansprüche 5 bis 7, **dadurch gekennzeichnet, dass** die Raum-Teilflächen (421) durch einen Polygonzug gebildet sind, der die zugehörige Ringraumfläche wenigstens teilweise begrenzt.

9. Schwenkgelenk nach einem der Ansprüche 1 bis 8, **dadurch gekennzeichnet, dass** die eine der den Ringraum (2) begrenzenden Ringraumflächen eine kreiszylindrische Mantelfläche ist.

10. Schwenkgelenk nach einem der Ansprüche 2 bis 9, **dadurch gekennzeichnet, dass** die Klemmelemente (61) des Klemmorgans (6) sich parallel zur Gelenkachse (100) erstreckende Zylinderelemente (611) sind.

11. Schwenkgelenk nach einem der Ansprüche 2 bis 10, **dadurch gekennzeichnet, dass** das die beiden Klemmelemente (61) eines genannten Klemmorgans (6) verbindende Distanzmittel (62) ein unter Kraft arbeitendes Kraftorgan (621) ist, wobei die die Klemmelemente (61) beabstandende Distanz (a) einer Kraft/Weg-Funktion unterliegt.

12. Schwenkgelenk nach Anspruch 11, **dadurch gekennzeichnet, dass** das Kraftorgan (621) ein in Umfangsrichtung (u) des Ringraums (2) wirkendes Federmittel ist.

13. Schwenkgelenk nach Anspruch 11 oder 12, **dadurch gekennzeichnet, dass** das Kraftorgan (621) die beiden zugehörigen Klemmelemente (61) mittels in Umfangsrichtung (u) des Ringraums (2) wirkender Kraft wenigstens in den einen der beiden Zustände, das heißt in den Haltezustand bzw. in den Freigabezustand, setzt und dass die Betätigungseinrichtung (7) Steuerelemente (72) aufweist, die zum Einstellen des Haltezustandes bzw. des Freigabezustandes an den Klemmelementen (61) angreifen und gegen die Kräfte der Kraftorgane (621) arbeiten.

14. Schwenkgelenk nach Anspruch 13, **dadurch gekennzeichnet, dass** wenigstens ein Steuerelement (72) wenigstens teilweise innerhalb des Ringraums (2) zwischen zwei benachbarten Klemmelementen (61) angeordnet ist.

15. Schwenkgelenk nach Anspruch 13 oder 14, **dadurch gekennzeichnet, dass** wenigstens einem Umfangsabschnitt (21) des Ringraums (2), welcher Umfangsabschnitt (21) frei von Distanzmitteln (62) der Klemmorgane (6) ist, ein genanntes Steuerelement (72) zugeordnet ist.

16. Schwenkgelenk nach einem der Ansprüche 13 bis 15, **dadurch gekenn- zeichnet,** dass wenigstens einem Umfangsabschnitt (22) des Ringraums (2), in dem das Distanzmittel (62) eines genannten Klemmorgans (6) angeordnet ist, ein genanntes Steuerelement (72) zugeordnet ist.

17. Schwenkgelenk nach einem der Ansprüche 13 bis 16, **dadurch gekennzeichnet, dass** wenigstens ein Distanzmittel eines genannten Klemmorgans durch ein genanntes Steuermittel gebildet ist.

18. Schwenkgelenk nach einem der Ansprüche 13 bis 17, **dadurch gekennzeichnet, dass** in dem Ringraum (2) wenigstens eine kettenartigen Reihe eingerichtet ist, die durch genannte Klemmelemente (61), genannte Distanzmittel (62) und genannte Steuerelemente (72) gebildet ist, wobei die Kettenglieder im Freigabezustand der Klemmorgane (6) miteinander in Verbindung stehen.

19. Schwenkgelenk nach einem der Ansprüche 1 bis 18, **dadurch gekennzeichnet, dass** zwischen dem Außenkörper (3) und dem Innenkörper (4) des Schwenkgelenks (1) ein Haltemittel angeordnet ist, das sämtliche Klemmorgane (6) mit zum Innenkörper (4) gerichteter Haltekraft beaufschlagt.

20. Schwenkgelenk nach Anspruch 19, **dadurch gekennzeichnet, dass** das Haltemittel durch ein um sämtliche Klemmorgane (6) gelegtes elastisches Spannmittel gebildet ist.,

21. Schwenkgelenk nach einem der Ansprüche 2 bis 20, **dadurch gekennzeichnet, dass** die Betätigungseinrichtung (7) wenigstens ein Steuerelement (72) aufweist, das zum Einstellen des Haltezustands bzw. des Freigabezustands an zugehörigen genannten Klemmorganen (6) angreift, wobei das Steuerelement (72) an dem Innenkörper (4) gelagert und quer zur Umfangsrichtung (u) des Ringraums (2) stellbewegbar angeordnet ist.

22. Schwenkgelenk nach Anspruch 21, **dadurch gekennzeichnet, dass** mehrere genannte Steuerelemente (72) mittels eines Steuerteils (73) stellbewegbar sind, das in einem Hohlraum des Innenkörpers (4) angeordnet und längs der Zylinderachse (40) des Innenkörpers (4) verstellbar ist.

23. Schwenkgelenk nach einem der Ansprüche 1 bis 22, **dadurch gekennzeichnet, dass** das Schwenkgelenk (1) mit mehreren genannten Klemmorganen (6) symmetrisch zur Gelenkachse (100) ausgebildet ist.

24. Schwenkgelenk nach einem der Ansprüche 1 bis 23, **dadurch gekennzeichnet, dass** das Schwenkgelenk (1) wenigstens sechs der Klemmorgane (6) umfasst, die über den Ringumfang des Ringraums (2) gleichmäßig verteilt angeordnet sind.

25. Schwenkgelenk nach einem der Ansprüche 1 bis 24, **dadurch gekennzeichnet, dass** der Außenkörper (3) und der Innenkörper (4) mittels außerhalb des Ringraums (2) eingerichteter Radiallager (11) drehbewegbar aneinander gelagert sind.

26. Halte- und Positioniereinrichtung (8), umfassend eine Reihe von miteinander verbundenen, jeweils eine Gelenkachse (100) aufweisenden Schwenkgelenken (1) und eine Steuereinrichtung (9) zum jeweils gleichzeitigen Freigeben und Blockieren der Schwenkbewegungen der Schwenkgelenke (1), **dadurch gekennzeichnet, dass** die Schwenkgelenke (1) jeweils durch ein Schwenkgelenk (1) nach einem der Ansprüche 1 bis 25 gebildet sind.

27. Halte- und Positioniereinrichtung (8) nach Anspruch 26, **dadurch gekennzeichnet, dass** die Steuereinrichtung (9) entsprechend der Anzahl der in Reihe miteinander verbundenen Schwenkgelenke (1) Steuerteile (73) umfasst, die jeweils durch einen eine Steuerkontur aufweisenden Steuerkörper (731) gebildet sind, wobei jeder zu einem Schwenkgelenk (1) zugehörige, in Richtung der zugehörigen Gelenkachse (100) axial stellbewegbare Steuerkörper (731) wenigstens eine Steuerfläche (74) aufweist, die entsprechend der axialen Stellposition des Steuerkörpers (731) wenigstens ein zugehöriges, quer zur Gelenkachse (100) bewegbares Steuerelement (72) des zugehörigen Schwenkgelenks (1) zum Freigeben bzw. Blockieren der Schwenkbewegung steuert.

## Claims

1. Bidirectional pivoting joint (1), comprising a hollow cylindrical outer body (3) having a hollow space (31), and a cylindrical inner body (4) arranged in the hollow space (31), wherein the outer body (3) and the inner body (4) can be rotated with coaxial cylinder axes (30, 40) relative to one another about a joint axis (100), and a locking device (5) which optionally locks and releases the relative rotational movement between the outer body (3) and the inner body (4), wherein the locking device (5) is provided by means of an annular space (2) which is concentric with the joint axis (100) and lies between the outer body (3) and the inner body (4) with radially narrowing and, by contrast, radially wider peripheral regions (201, 202) in an alternating arrangement between the annular space surfaces of the annular space (2), specifically between an inner surface (32) of the outer body (3) and an outer surface (42) of the inner body (4), at least two clamping members (6) distributed over the annular periphery of the annular space (2) and arranged therein, said clamping members each having a cross-section being variable in the peripheral direction (u) of the annular space (2), and an actuating device (7) setting the aforementioned variable cross-sections of the clamping members (6), wherein each clamping member (6) assumes two states, namely a holding state determined by a first said cross-section of each clamping member (6) in which each clamping member (6) lies in form-fitting connection against the inner surface (32) of the outer body (3) and against the outer surface (42) of the inner body (4) in said narrowing peripheral regions (201) which adjoin one another, so that a self-holding clamping connection is established capturing the clamping members (6) between the inner surface (32) and the outer surface (42), blocking the relative rotational movement between the outer body (3) and the inner body (4) in each rotation direction (d), and namely a releasing state determined by a second said cross-section of each clamping member (6) in which the clamping connection is released in order to free relative rotational movement and wherein the actuating device (7) comprises an actuating means (71) which acts on the clamping members (6) to establish, firstly, the holding state and, secondly, the releasing state, **characterized in that** the actuating means (71) is configured to establish the releasing state by means of a change of the length of the clamping member (6) in the circumferential direction (u) of the annular space (2), such that the clamping members (6) comes free from the form-fitting connection on both sides in the circumferential direction (u) of the annular space (2), whereby the rotational movement between the outer body (3) and the inner body (4) is released bidirectionally.

2. Pivoting joint according to claim 1, **characterised in that** at least one said clamping member (6) is formed by two clampable clamping elements (61) arranged offset in the peripheral direction (u) of the annular space (2) and a spacing means (62) having a length which is changeable in the peripheral direction (u) of the annular space (2), said spacing means spacing the two clamping elements (61) apart from one another in the peripheral direction (u) of the annular space (2) and displacing said clamping elements (61) relative to one another to establish at least one of the two positions, that is the holding position or the releasing position.

3. Pivoting joint according to claim 1 or 2, **characterised in that** the annular space (2) is subdivided into peripheral sections (20), each accommodating one said clamping member (6), wherein each peripheral section (20) has two of the narrowing peripheral regions (201).

4. Pivoting joint according to claim 3, **characterised in that** each clamping member (6) comprises a pair of clamping elements (61) which, in order to establish the holding state, each reach into the two narrowing peripheral regions (201) of a said peripheral section (20).

5. Pivoting joint according to claim 4, **characterised in that** an annular space surface of the annular space (2), that is the outer surface (42) of the inner body (4) or the inner surface (32) of the outer body (3) is formed by space partial surfaces (421) which determine said peripheral sections (20) of the annular space (2) and, together with the other annular space surface of the annular space (2), form the narrowing peripheral regions (201).

6. Pivoting joint according to claim 5, **characterised in that** the two clamping elements (61) of a clamping member (6) are each arranged on a space partial surface (421) of two adjacent said space partial surfaces (421).

7. Pivoting joint according to claim 5 or 6, **characterised in that** the two clamping elements (61) of a clamping member (6) are arranged on a common said space partial surface (421).

8. Pivoting joint according to any one of claims 5 to 7, **characterized in that** the space partial surfaces (421) are formed by a polygonal line which at least partially borders the associated annular space surface.

9. Pivoting joint according to any one of claims 1 to 8, **characterized in that** one of the annular space surfaces bordering the annular space (2) is a circle cylindrical shell surface.

10. Pivoting joint according to any one of claims 2 to 9, **characterized in that** the clamping elements (61) of the clamping member (6) are cylinder elements (611) extending parallel to the joint axis (100).

11. Pivoting joint according to any one of claims 2 to 10, **characterized in that** the spacing means (62) connecting the two clamping elements (61) of a said clamping member (6) is an actuator (621) operating under force, wherein the spacing (a) separating the clamping elements (61) is subject to a force-deflection function.

12. Pivoting joint according to claim 11, **characterized in that** the actuator (621) is a spring means acting in the peripheral direction (u) of the annular space (2).

13. Pivoting joint according to claim 11 or 12, **characterized in that** the actuator (621) places the two associated clamping elements (61) into at least one of the two states by means of the force acting in the peripheral direction (u) of the annular space (2), that is, into the holding state or into the releasing state and that the actuating device (7) has control elements (72) which engage with said clamping elements (61) in order to establish the holding state or the releasing state and act against the forces of the actuators (621).

14. Pivoting joint according to claim 13, **characterized in that** at least one control element (72) is arranged at least partially within the annular space (2) between two adjacent clamping elements (61).

15. Pivoting joint according to claim 13 or 14, **characterized in that** said control element (72) is associated with at least one peripheral section (21) of the annular space (2), said peripheral section (21) being free from spacing means (62) of the clamping members (6).

16. Pivoting joint according to any one of claims 13 to 15, **characterized in that** at least one peripheral section (22) of the annular space (2) in which the spacing means (62) of a said clamping member (6) is arranged, is associated with a said control element (72).

17. Pivoting joint according to any one of claims 13 to 16, **characterized in that** at least one spacing means of a said clamping member is formed by a said control element.

18. Pivoting joint according to any one of claims 13 to 17, **characterized in that** at least one chain-like series formed by said clamping elements (61), said spacing means (62) and said control elements (72) is arranged in the annular space (2), wherein in the releasing state of the clamping members (6), the chain members are linked to one another.

19. Pivoting joint according to any one of claims 1 to 18, **characterized in that** arranged between the outer body (3) and the inner body (4) of the pivoting joint (1) is a holding means which applies to all the clamping members (6) a holding force directed toward the inner body (4).

20. Pivoting joint according to claim 19, **characterized in that** the holding means is formed by an elastic tensioning means laid round all the clamping members (6).

21. Pivoting joint according to any one of claims 2 to 20, **characterized in that** the actuating device (7) has at least one control element (72) which engages in associated said clamping members (6) to establish the holding state or the releasing state, wherein the control element (72) is mounted on the inner body (4) and is arranged actuatable transversely to the peripheral direction (u) of the annular space (2).

22. Pivoting joint according to claim 21, **characterized in that** a plurality of said control elements (72) is actuatable by means of a control part (73) which is arranged within a hollow space of the inner body (4) and is displaceable along the cylinder axis (40) of the inner body (4).

23. Pivoting joint according to any one of claims 1 to 22, **characterized in that** the pivoting joint (1) is configured with a plurality of said clamping members (6) symmetrical to the joint axis (100).

24. Pivoting joint according to any one of claims 1 to 23, **characterized in that** the pivoting joint (1) comprises at least six of the clamping members (6) which are arranged evenly distributed over the annular periphery of the annular space (2).

25. Pivoting joint according to any one of claims 1 to 24, **characterized in that** the outer body (3) and the inner body (4) are rotatably mounted on one another by means of radial bearings (11) arranged outside the annular space (2).

26. Holding and positioning device (8), comprising a series of mutually connected pivoting joints (1), each having a joint axis (100) and a control device (9) for simultaneous releasing and blocking of the pivoting movements of the pivoting joints (1) in each case, **characterised in that** the pivoting joints (1) are each formed by a pivoting joint (1) according to any one of claims 1 to 25.

27. Holding and positioning device (8) according to claim 26, **characterised in that** the control device (9) comprises control parts (73) corresponding to the number of pivoting joints (1) connected in series to one another, said control parts each being formed by a control body (731) having at least one control contour, wherein each control body (731) which is axially actuatable in the direction of the associated joint axis (100) and belongs to a pivoting joint (1), has at least one control surface (74) which, according to the axial actuation position of the control body (731), controls at least one associated control element (72) which is movable transversely to the joint axis (100) for releasing or blocking the pivoting movement.

## Revendications

1. Une articulation pivotante bidirectionnelle (1), comprenant un corps extérieur cylindrique (3) creux muni d'une cavité (31) et un corps intérieur cylindrique (4) disposé dans la cavité (31), dans laquelle le corps extérieur (3) et le corps intérieur (4) pivotent l'un par rapport à l'autre au moyen d'axes de cylindre coaxiaux (30, 40) et d'un axe d'articulation (100), ainsi qu'un dispositif de blocage (5) qui bloque ou débloque sélectivement le mouvement de rotation entre le corps extérieur (3) et le corps intérieur (4), le dispositif de blocage (5) étant conçu d'un espace annulaire (2) concentrique par rapport à l'axe d'articulation (100) entre le corps extérieur (3) et le corps intérieur (4), avec entre les faces de l'espace annulaire (2), en particulier entre une surface intérieure (32) du corps extérieur (3) et une surface extérieure (42) du corps intérieur (4) des zones périphériques radialement rétrécies et radialement plus larges (201, 202) se succédant en alternance comprenant au moins deux organes de serrage (6) sur le pourtour circulaire de l'espace annulaire (2) réparties dans ces dernières et pouvant être modifiés respectivement dans le sens périphérique (u) de l'espace annulaire (2), ainsi qu'un dispositif d'actionnement (7) s'ajustant sur ladite section transversale modifiable de l'organe de serrage (6), dans laquelle chaque organe de serrage (6) prend deux états, notamment un état de retenue défini par une première section transversale donnée de chaque organe de serrage (6) dans lequel chaque organe de serrage (6) se trouve en appui dans les zones périphériques rétrécies données (201) contre la surface intérieure (32) du corps extérieur (3) et la surface extérieure (42) du corps intérieur (4) en exerçant une liaison par serrage, les organes de serrage (6) se trouvant entre la surface intérieure (32) et la surface extérieure (42) établissant ainsi une liaison par serrage à auto-retenue bloquant dans chaque direction de rotation (d) le mouvement rotatif relatif entre le corps extérieur (3) et le corps intérieur (4), et un état d'actionnement définit par une seconde section transversale donnée de chaque organe de serrage (6), dans lequel la liaison par serrage est supprimée pour débloquer le mouvement rotatif relatif, et dans lequel le dispositif d'actionnement (7) comprend un moyen d'actionnement (71) agissant sur l'organe de blocage (6) pour régler d'une part l'état de retenue et d'autre part l'état d'actionnement, **caractérisée en ce que** le moyen d'actionnement (71) de réglage de l'état d'actionnement par la modification de la longueur de l'organe de serrage (6) dans le sens périphérique (u) de l'espace annulaire (2) est réglé de façon à ce que les organes de serrage (6) dans le sens périphérique (u) de l'espace annulaire (2) se libèrent de la liaison par complémentarité des formes, débloquant ainsi le mouvement rotatif entre le corps extérieur (3) et le corps intérieur (4).

2. Une articulation pivotante selon la revendication 1, **caractérisée en ce que** au moins un des organes de serrage donnés (6) est constitué de deux éléments de serrage (61) pouvant être bloqués et disposés de façon décalée dans le sens périphérique (u) de l'espace annulaire (2) et d'un moyen d'écartement (62) modifiable en longueur dans le sens périphérique (u) de l'espace annulaire (2), écartant les deux éléments de serrage (61) l'un de l'autre dans le sens périphérique (u) de l'espace annulaire (2) et déplaçant l'un par rapport à l'autre pour régler au moins l'une des deux positions, à savoir la position de retenue ou la position d'actionnement.

3. Une articulation pivotante selon la revendication 1 ou 2, **caractérisée en ce que** l'espace annulaire (2) est subdivisé en parties périphériques (20) hébergeant respectivement un organe de serrage (6) donné, dans laquelle chaque partie périphérique (20) comprend deux des zones périphériques rétrécies (201).

4. Une articulation pivotante selon la revendication 3, **caractérisée en ce que** chaque organe de serrage (6) comprend une paire d'éléments de serrage (61) entourant respectivement une partie périphérique (20) donnée dans les deux zones périphériques rétrécies (201) afin de produire l'état de retenue.

5. Une articulation pivotante selon la revendication 4, **caractérisée en ce que** la surface annulaire de l'espace annulaire (2), à savoir la surface extérieure (42) du corps intérieur (4) ou la surface intérieure (32) du corps extérieur (3), est composée de surfaces partielles de l'espace (421) définissant les parties périphériques (20) données de l'espace annulaire (2), et constituant les zones périphériques rétrécies (201) en collaboration avec les autres surfaces annulaires de l'espace annulaire (2).

6. Une articulation pivotante selon la revendication 5, **caractérisée en ce que** les deux éléments de serrage (61) d'un organe de serrage (6) sont disposés respectivement contre la surface partielle de l'espace de deux surfaces partielles de l'espace (421) données adjacentes.

7. Une articulation pivotante selon la revendication 5 ou 6, **caractérisée en ce que** les deux éléments de serrage (61) d'un organe de serrage (6) sont disposés contre une surface partielle de l'espace (421) donnée commune.

8. Une articulation pivotante selon l'une des revendications 5 à 7, **caractérisée en ce que** les surfaces partielles de l'espace (421) sont constituées d'un polygone limitant au moins partiellement la surface annulaire correspondante.

9. Une articulation pivotante selon l'une des revendications 1 à 8, **caractérisée en ce que** l'une des surfaces annulaires limitant l'espace annulaire (2) est une surface d'enveloppe cylindrique circulaire.

10. Une articulation pivotante selon l'une des revendications 2 à 9, **caractérisée en ce que** les éléments de serrage (61) de l'organe de serrage (6) sont des éléments cylindriques (611) s'étendant parallèlement à l'axe d'articulation (100)

11. Une articulation pivotante selon l'une des revendications 2 à 10, **caractérisée en ce que** les deux éléments de serrage (61) d'un organe de serrage (6) donné sont des moyens d'écartement (62) de liaison d'un organe de puissance (621) fonctionnant sous l'effet de la puissance, dans lequel la distance d'écartement (a) par rapport aux éléments de serrage (61) répond à un rapport puissance/déplacement.

12. Une articulation pivotante selon la revendication 11, **caractérisée en ce que** l'organe de puissance (621) est un moyen élastique fonctionnant dans le sens périphérique (u) de l'espace annulaire (2).

13. Une articulation pivotante selon la revendication 11 ou 12, **caractérisée en ce que** l'organe de puissance (621) place les deux éléments de serrage (61) correspondants dans au moins l'un des deux états, à savoir dans l'état de retenue ou dans l'état d'actionnement, sous l'effet d'une puissance exercée dans le sens périphérique (u) de l'espace annulaire (2) et **en ce que** le dispositif d'actionnement (7) comprend des éléments de commande (72) s'engageant contre les éléments de serrage (61) et fonctionnant contre les forces de l'organe de puissance (621) pour régler l'état de retenue ou l'état d'actionnement.

14. Une articulation pivotante selon la revendication 13, **caractérisée en ce qu'**au moins un élément de commande (72) est disposé au moins partiellement dans l'espace annulaire (2) entre deux éléments de serrage (61) adjacents.

15. Une articulation pivotante selon la revendication 13 ou 14, **caractérisée en ce qu'**au moins une section périphérique (21) de l'espace annulaire (2) est assignée à un élément de commande (72) donné, ladite section périphérique (21) étant dépourvue de moyens d'écartement (62) des organes de serrage (6).

16. Une articulation pivotante selon la revendication 13 ou 15, **caractérisée en ce que** au moins une section périphérique (22) de l'espace annulaire (2), dans laquelle se trouve le moyen d'écartement (62) d'un organe de serrage (6) donné, est assignée à un élément de commande (72) donné.

17. Une articulation pivotante selon l'une des revendications 13 à 16, **caractérisée en ce qu'**au moins un moyen d'écartement d'un organe de serrage donné est constitué d'un moyen de commande donné.

18. Une articulation pivotante selon l'une des revendications 13 à 17, **caractérisée en ce que** l'espace annulaire (2) comprend au moins une rangée d'éléments en chapelet constituée des éléments de serrage (61) donnés, des moyens d'écartement (62) donnés et des éléments de commande (72) donnés, dans laquelle les éléments de la chaîne sont liés les uns aux autres lorsque l'organe de serrage (6) se trouve en état d'actionnement.

19. Une articulation pivotante selon l'une des revendications 1 à 18, **caractérisée en ce que** un moyen de retenue est disposé entre le corps extérieur (3) et le corps intérieur (4) de l'articulation pivotante (1) afin d'appliquer la force de retenue exercée sur le corps intérieur (4) à l'ensemble des organes de serrage (6).

20. Une articulation pivotante selon la revendication 19, **caractérisée en ce que** le moyen de retenue est constitué d'un moyen de serrage élastique posé autour de l'ensemble des organes de serrage (6).

21. Une articulation pivotante selon l'une des revendications 2 à 20, **caractérisée en ce que** le dispositif d'actionnement (7) comprend au moins un élément de commande (72) s'engageant contre les organes de serrage (6) donnés correspondants pour régler l'état de retenue ou l'état d'actionnement, dans laquelle l'élément de commande (72) est placé contre le corps intérieur (4) et disposé de façon transversale par rapport au sens périphérique (u) de l'espace annulaire (2) de manière à pouvoir se déplacer pour effectuer des réglages.

22. Une articulation pivotante selon la revendication 21, **caractérisée en ce que** plusieurs éléments de commande (72) donnés peuvent être déplacés pour effectuer des réglages à l'aide de composants de commande (73), disposés dans une cavité du corps intérieur (4) et pouvant être déplacés le long de l'axe de cylindre (40) du corps intérieur (4).

23. Une articulation pivotante selon l'une des revendications 1 à 22, **caractérisée en ce que** l'articulation pivotante (1) est conçue de façon symétrique à l'axe d'articulation (100) avec plusieurs organes de serrage (6) donnés.

24. Une articulation pivotante selon l'une des revendications 1 à 23, **caractérisée en ce que** l'articulation pivotante (1) comprend au moins six organes de serrage (6), répartis de façon égale sur le pourtour circulaire de l'espace annulaire (2).

25. Une articulation pivotante selon l'une des revendications 1 à 24, **caractérisée en ce que** le corps extérieur (3) et le corps intérieur (4) sont montés l'un sur l'autre de façon pivotante par l'intermédiaire d'un palier radial (11) disposé hors de l'espace annulaire (2).

26. Un dispositif de retenue et de positionnement (8), comprenant une rangée d'articulations pivotantes (1) liées les unes aux autres et munies respectivement d'un axe d'articulation (100) et un dispositif de commande (9) assurant respectivement l'activation et la retenue simultanée des mouvements de pivotement de l'articulation pivotante (1), **caractérisé en ce que** l'articulation pivotante (1) respective est constituée d'une articulation pivotante (1) selon l'une des revendications 1 à 25.

27. Un dispositif de retenue et de positionnement (8) selon la revendication 26, **caractérisé en ce que** le dispositif de commande (9) comprend des éléments de commande (73) constitués respectivement d'un corps de commande (731) muni d'un contour de commande, en fonction du nombre d'articulations pivotantes (1) liées les unes aux autres dans la rangée, dans lequel chaque corps de commande (731) appartenant à une articulation pivotante (1) pouvant être déplacé de façon axiale en direction de l'axe d'articulation (100) correspondant comprend au moins une surface de commande (74) pilotant au moins un élément de commande (72) pouvant être déplacé de façon transversale par rapport à l'axe d'articulation (100) de l'articulation pivotante (1) correspondante, en fonction de la position de réglage du corps de commande (731), afin d'assurer l'actionnement ou le blocage des mouvements de pivotement.
